# EUROPEAN PATENT SPECIFICATION

(11) **EP 1 910 563 B1**
(45) Date of publication and mention of the grant of the patent: **21.04.2010**
(21) Application number: 06757809.6
(22) Date of filing: 23.06.2006
(51) Int. Cl.: C12Q 1/68

(54) **IMPROVED STRATEGIES FOR SEQUENCING COMPLEX GENOMES USING HIGH THROUGHPUT SEQUENCING TECHNOLOGIES**
VERBESSERTE STRATEGIEN ZUR SEQUENZIERUNG KOMPLEXER GENOME UNTER VERWENDUNG VON SEQUENZIERTECHNIKEN MIT HOHEM DURCHSATZ
STRATÉGIES AMÉLIORÉES POUR LE SÉQUENÇAGE DE GÉNOMES COMPLEXES UTILISANT DES TECHNIQUES DE SÉQUENÇAGE À HAUT RENDEMENT

(30) Priority: 23.06.2005 US 693053 P; 08.09.2005 US 714897 P; 16.01.2006 EP 06075104; 17.01.2006 US 759034 P
(43) Date of publication of application: 16.04.2008
(73) Proprietor: KEYGENE N.V., 6708 PW Wageningen (NL)
(72) Inventor: VAN EIJK, Michael, Josephus, Theresia, NL-5373 EJ Herpen (NL); SØRENSEN, Anker, Preben, NL-6871 HZ Renkum (NL); HOGERS, René, Cornelis, Josephus, NL-6715 GR Ede (NL)
(74) Representative: de Lang, Robbert-Jan
(86) International application number: PCT/NL2006/000312
(87) International publication number: WO 2006/137734

(56) References cited:
- EP-A- 0 534 858
- EP-A- 1 124 990
- WO-A-00/61800
- WO-A-01/88189
- WO-A-98/51789
- WO-A-2005/003375
- US-A1- 2005 064 406
- US-B1- 6 534 293
- VOS P ET AL: "AFLP: A NEW TECHNIQUE FOR DNA FINGERPRINTING" NUCLEIC ACIDS RESEARCH, OXFORD UNIVERSITY PRESS, SURREY, GB, vol. 23, no. 21, 1995, pages 4407-4414, XP000939214 ISSN: 0305-1048 cited in the application

## Description

### Technical Field

The present invention relates to the fields of molecular biology and genetics. The invention relates to improved strategies for determining the sequence of, preferably complex (i.e. large) genomes, based on the use of high throughput sequencing technologies.

### Background of the invention

Assembly of whole genome shotgun sequences of large genomes (from 100 Mbp upwards) to draft genome sequences is a complex issue. Many plants and animals further contain a large number of repeat sequences, thereby further complicating the problem. This computational problem is further enlarged by the emergence of high throughput sequencing technologies, such as by technologies of 454 Life Science. These technologies are often no longer based on Sanger dideoxysequencing, but predominantly on sequencing by synthesis (pyrosequencing), which is easier to perform on a solid surface. Sequencing by synthesis provides a large amount of sequences, albeit of a relative short length (about 100 bp) compared to the relatively large length of 500 to 1000 bp as is common for Sanger dideoxysequencing.

On of the disadvantages of such short fragments is that the assembly of contigs to determine the genome sequence requires enormous computational power, making the current methods of sequencing a relatively expensive and time consuming quest. Consequently there is a need for cheap, reliable and fast methods of sequencing complex, i.e. large genomes to further the technology to what is sometimes called the "1000$ genome", i.e. a method that allows the determination of the entire sequence of a complex genome (human in particular) for not more than 1000$. This would allow i.a. for the development of personalized medication.

### Summary of the invention

The present inventors have now found that with a different strategy this problem can be solved and the high throughput sequencing technologies can be efficiently used in genome assembly.

The invention comprises employing a technology that divides the genome in reproducible and complementary parts by restricting the genome with one or more restriction endonucleases to yield a set of restriction fragments and subsequently providing a subset of restriction fragments by selective amplification. The subset is sequenced and assembled to a contig. By repeating this step for one or more different sets of restriction endonucleases, different contigs are obtained. These different contigs are used to assemble the draft genome sequence. The invention does not require any knowledge of the sequence and can be applied to genomes of any size and complexity. The invention can be scaled up for any type and size of the genome. The present invention provides a quicker, reliable and faster access to any genome of interest and thereby provides for accelerated analysis of the genome.

### Definitions

In the following description and examples a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this invention belongs.

Nucleic acid: a nucleic acid according to the present invention may include any polymer or oligomer of pyrimidine and purine bases, preferably cytosine, thymine, and uracil, and adenine and guanine, respectively (*See* Albert L. Lehninger, Principles of Biochemistry, at 793-800 (Worth Pub. 1982)). The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogenous or homogenous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

Complexity reduction: the term complexity reduction is used to denote a method wherein the complexity of a nucleic acid sample, such as genomic DNA, is reduced by the generation of a subset of the sample. This subset can be representative for the whole (i.e. complex) sample and is preferably a reproducible subset. Reproducible means in this context that when the same sample is reduced in complexity using the same method, the same, or at least comparable, subset is obtained. The method used for complexity reduction may be any method for complexity reduction known in the art. Examples of methods for complexity reduction include for example AFLP® (Keygene N.V., the Netherlands; see e.g. EP 0 534 858), the methods described by Dong (see e.g. WO 03/012118, WO 00/24939, EP 1 124 990) or Barany (e.g. US 6 534 293), indexed linking (Unrau et al., vide infra), etc. The complexity reduction methods used in the present invention have in common that they are reproducible. Reproducible in the sense that when the same sample is reduced in complexity in the same manner, the same subset of the sample is obtained, as opposed to more random complexity reduction such as microdissection or the use of mRNA (cDNA) which represents a portion of the genome transcribed in a selected tissue and for its reproducibility is depending on the selection of tissue, time of isolation etc..

Tagging: the term tagging refers to the addition of a tag to a nucleic acid sample in order to be able to distinguish it from a second or further nucleic acid sample. Tagging can e.g. be performed by the addition of a sequence identifier during complexity reduction or by any other means known in the art. Such sequence identifier can e.g. be a unique base sequence of varying but defined length uniquely used for identifying a specific nucleic acid sample. Typical examples thereof are for instance ZIP sequences. Using such tag, the origin of a sample can be determined upon further processing. In case of combining processed products originating from different nucleic acid samples, the different nucleic acid samples should be identified using different tags.

Tagged library: the term tagged library refers to a library of tagged nucleic acid.

Sequencing: The term sequencing refers to determining the order of nucleotides (base sequences) in a nucleic acid sample, e.g. DNA or RNA.

Aligning and alignment: With the term "aligning" and "alignment" is meant the comparison of two or more nucleotide sequence based on the presence of short or long stretches of identical or similar nucleotides. Several methods for alignment of nucleotide sequences are known in the art, as will be further explained below. Sometimes assembling is used as a synonym.

High-throughput screening: High-throughput screening, often abbreviated as HTS, is a method for scientific experimentation especially relevant to the fields of biology and chemistry. Through a combination of modern robotics and other specialised laboratory hardware, it allows a researcher to effectively screen large amounts of samples simultaneously.

Restriction endonuclease: a restriction endonuclease or restriction enzyme is an enzyme that recognizes a specific nucleotide sequence (target site) in a double-stranded DNA molecule, and will cleave both strands of the DNA molecule at every target site.

Restriction fragments: the DNA molecules produced by digestion with a restriction endonuclease are referred to as restriction fragments. Any given genome (or nucleic acid, regardless of its origin) will be digested by a particular restriction endonuclease into a discrete set of restriction fragments. The DNA fragments that result from restriction endonuclease cleavage can be further used in a variety of techniques and can for instance be detected by gel electrophoresis.

Gel electrophoresis: in order to detect restriction fragments, an analytical method for fractionating double-stranded DNA molecules on the basis of size can be required. The most commonly used technique for achieving such fractionation is (capillary) gel electrophoresis. The rate at which DNA fragments move in such gels depends on their molecular weight; thus, the distances travelled decrease as the fragment lengths increase. The DNA fragments fractionated by gel electrophoresis can be visualized directly by a staining procedure e.g. silver staining or staining using ethidium bromide, if the number of fragments included in the pattern is sufficiently small. Alternatively further treatment of the DNA fragments may incorporate detectable labels in the fragments, such as fluorophores or radioactive labels.

Ligation: the enzymatic reaction catalyzed by a lipase enzyme in which two double-stranded DNA molecules are covalently joined together is referred to as ligation. In general, both DNA strands are covalently joined together, but it is also possible to prevent the ligation of one of the two strands through chemical or enzymatic modification of one of the ends of the strands. In that case the covalent joining will occur in only one of the two DNA strands.

Synthetic oligonucleotide: single-stranded DNA molecules having preferably from about 10 to about 50 bases, which can be synthesized chemically are referred to as synthetic oligonucleotides. In general, these synthetic DNA molecules are designed to have a unique or desired nucleotide sequence, although it is possible to synthesize families of molecules having related sequences and which have different nucleotide compositions at specific positions within the nucleotide sequence. The term synthetic oligonucleotide will be used to refer to DNA molecules having a designed or desired nucleotide sequence.

Adaptors: short double-stranded DNA molecules with a limited number of base pairs, e.g. about 10 to about 30 base pairs in length, which are designed such that they can be ligated to the ends of restriction fragments. Adaptors are generally composed of two synthetic oligonucleotides which have nucleotide sequences which are partially complementary to each other. When mixing the two synthetic oligonucleotides in solution under appropriate conditions, they will anneal to each other forming a double-stranded structure. After annealing, one end of the adaptor molecule is designed such that it is compatible with the end of a restriction fragment and can be ligated thereto; the other end of the adaptor can be designed so that it cannot be ligated, but this need not be the case (double ligated adaptors).

Adaptor-ligated restriction fragments: restriction fragments that have been capped by adaptors as a result of ligation.

Primers: in general, the term primers refers to a DNA strand which can prime the synthesis of DNA. DNA polymerase cannot synthesize DNA de novo without primers: it can only extend an existing DNA strand in a reaction in which the complementary strand is used as a template to direct the order of nucleotides to be assembled. We will refer to the synthetic oligonucleotide molecules which are used in a polymerase chain reaction (PCR) as primers.

DNA amplification: the term DNA amplification will be typically used to denote the in vitro synthesis of double-stranded DNA molecules using PCR. It is noted that other amplification methods exist and they may be used in the present invention without departing from the gist thereof.

### Detailed description of the invention

The present invention provides for a method for determining a genome sequence comprising the steps of:
(a) providing a first subset of the genome by digesting the genome with at least one first restriction endonuclease to provide restriction fragments;
(b) ligating at least one adaptor to the restriction fragments of the first subset to provide a first set of adaptor-ligated restriction fragments;
(c) selectively amplifying the first set of adaptor-ligated restriction fragments using a first primer combination wherein at least a first primer contains a section that is complementary to the adaptor and to part of the recognition sequence of the restriction endonuclease and that further contains a first selected sequence at the 3' end of the primer sequence, wherein the first selected sequence comprises 1-10 selective nucleotides, to provide a first subset of amplified adaptor-ligated restriction fragments;
(d) repeating step (c) with at least a second and/or further primer combination(s) wherein the primer contains a different second and/or further selected sequence at its 3'end that contains the same number of selective nucleotides, to provide for second and/or further subsets of amplified adaptor-ligated restriction fragments;
(e) fragmenting each of the first, second and/or further subsets of amplified adaptor-ligated restriction fragments, optionally followed by size selection fragments in the optimal size range, to generate first, second and/or further sequencing libraries, followed by optional pooling of the libraries;
(f) determine (at least part of) the nucleotide sequence of (at least part of) the fragments contained in each of the first, second and/or further sequence libraries;
(g) aligning the sequence of the fragments in each of the first, second and/or further libraries to generate contigs of the amplified adaptor-ligated restriction fragments derived from the subset(s) of the genome:
(h) repeating steps (a)-(g) for at least one second and /or further restriction endonucleases;
(i) aligning the contigs obtained in step (g) and (h) for each of the second and /or further restriction endonucleases to provide for a sequence of the genome.

In step (a) of the method, the genome of interested is subjected to one or more restriction endonucleases. In certain embodiments, at least two restriction endonucleases are used. In certain embodiments, in particular with large genomes, three or more restriction endonucleases can be used. Digestion of the genome provides a first subset of the genome. The restriction endonucleases can be frequent cutters (i.e. typically 4 and 5 cutters, i.e. restriction endonucleases that have a recognition sequence of 4 or 5 nucleotides, respectively) or may be rare cutters, (i.e: typically 6 and higher cutters (7, 8, ... etc., i.e. restriction endonucleases that have a recognition sequence of 6 or more nucleotides, respectively), or combinations thereof. In certain embodiments a combination of a rare and frequent cutter is used. In certain embodiments two rare cutters may be used. The restriction endonucleases can be of any type, including IIs and IIsa types that cut the DNA outside their recognition sequence, either on one or on both sides of the recognition sequence.

In step (b) of the method, at least one adaptor is ligated to the restriction fragments obtained in step (a). Preferably, the adaptors are such that the restriction site is not restored upon ligation of the adaptor. It is also possible, for instance in case of two or more restriction endonucleases to employ two or more different adaptors. This ligation step yields adaptor- ligated restriction fragments. The adaptors, depending on the restriction endonuclease, can be blunt ended or may contain an overhang.

In certain embodiments, the adaptor may be a set of adaptors known as indexing linkers (Unrau et al., 1994, Gene, 145:163-169).

In step (c), the first set of adaptor-ligated restriction fragments is amplified using a first primer combination. The primer combination comprises at least a first primer that contains a section that is complementary to (at least part of) the adaptor and to part of the recognition sequence of the restriction endonuclease used in the restriction of the genome. Typically, the part of the recognition sequence is that part that remains after restriction of the sequence with the restriction endonuclease. At its 3'-end the primer contains a first selected sequence. The first selected sequence comprises a previously selected set of 1-10 nucleotides, preferably 1-8 selected nucleotides, preferably 1-5, more preferably 1-3. Such a primer may have the following, illustrative, structure (for 2 selective nucleotides (AC)) "5'-adaptor specific region-restriction sequence specific region-AC-3' ". This exemplary first primer thus contains 2 selective nucleotides AC which will only amplify adaptor-ligated fragments that contain the complementary TG as the first two nucleotides derived from the sequence of the restriction fragment. This provides the first subset of amplified adaptor-ligated restriction fragments.

The first primer combination may also comprise two selective primers, each carrying a selected sequence at their 3'-end. The primers can be tagged to allow for pooling strategies.

The amplification is preferably carried out using PCR. In certain embodiments the use of Long-Range PCR is preferred.

In step (d), the selective amplification is repeated with second and further primer combinations. At least one of the primers in each of the further primer combinations contains a different selected sequence at its 3'-end. The selection of the selected sequences is such that, given the number of selected nucleotides, all possible permutations of the selective nucleotides are used. In the above example this means AT, AG, AA, CA, CT, CG, CA etc. In practice this means that all adaptor-ligated restriction fragments within the subset of the genome (i.e. within the set of restriction fragments obtained using the one or more restriction endonucleases) haven been amplified.

In a preferred embodiment of the invention, the reduction of the complexity of the genome by selective amplification is performed by means of AFLP® (Keygene N.V., the Netherlands; *see* e.g. EP 0 534 858 and Vos et al. (1995). AFLP: a new technique for DNA fingerprinting, Nucleic Acids Research, vol. 23, no. 21, 4407-4414).

AFLP is a method for selective restriction fragment amplification. AFLP does not require any prior sequence information and can be performed on any starting DNA. In general, AFLP comprises the steps of:
(a) digesting a nucleic acid, in particular a DNA, with one or more specific restriction endonucleases, to fragment the DNA into a corresponding series of restriction fragments;
(b) ligating the restriction fragments thus obtained with a double-stranded synthetic oligonucleotide adaptor, one end of which is compatible with one or both of the ends of the restriction fragments, to thereby produce adaptor-ligated, preferably tagged, restriction fragments of the starting DNA;
(c) contacting the adaptor-ligated, preferably tagged, restriction fragments under hybridizing conditions with one or more oligonucleotide primers that contain selective nucleotides at their 3'-end;
(d) amplifying the adaptor-ligated, preferably tagged, restriction fragment hybridized with the primers by PCR or a similar technique so as to cause further elongation of the hybridised primers along the restriction fragments of the starting DNA to which the primers hybridised; and
(e) detecting, identifying or recovering the amplified or elongated DNA fragment thus obtained.

AFLP thus provides a reproducible subset of adaptor-ligated fragments. One useful variant of the AFLP technology uses no selective nucleotides (i.c. +0/+0 primers) and is sometimes called linker PCR. This also provides for a very suitable complexity reduction, in particular for smaller genomes.

For a further description of AFLP, its advantages, its embodiments, as well as the techniques, enzymes, adaptors, primers and further compounds and tools used therein, reference is made to US 6,045,994, EP-B-0 534 858, EP 976835 and EP 974672, WO01/88189 and Vos et al. Nucleic Acids Research, 1995, 23, 4407-4414.

Thus, in a preferred embodiment of the method of the present invention, the genome is reduced in complexity by
(a) digesting the nucleic acid sample with at least one restriction endonuclease to fragment it into restriction fragments;
(b) ligating the restriction fragments obtained with at least one double-stranded synthetic oligonucleotide adaptor having one end compatible with one or both ends of the restriction fragments to produce adaptor-ligated restriction fragments;
(c) contacting said adaptor-ligated restriction fragments with one or more oligonucleotide primers under hybridizing conditions; and
(d) amplifying said adapted restriction fragments by elongation of the one or more oligonucleotide primers,
(e) wherein at least one of the one or more oligonucleotide primers include a nucleotide sequence having the same nucleotide sequence as the terminal parts of the strands at the ends of said adapted restriction fragments, including the nucleotides involved in the formation of the target sequence for said restriction endonuclease and including at least part of the nucleotides present in the adaptors, wherein, optionally, at least one of said primers includes at its 3' end a selected sequence comprising at least one nucleotide located immediately adjacent to the nucleotides involved in the formation of the target sequence for said restriction endonuclease.

AFLP is a highly reproducible method for complexity reduction and is therefore particularly suited for the method according to the present invention.

Hitherto in the art of sequencing technology, the use of this selective amplification in the sequence determination of whole genomes, and in particular in complex genomes has not been disclosed or suggested. The AFLP-technology is known in the art as a fingerprinting technology and has not yet been identified as a solution to aid in the sequencing of complex genomes. In particular, the use of a set of primer combinations that cover all or most of the permutations of nucleotides for a given number of selective nucleotides (for instance 16 primer combinations in the case of two selective nucleotides) provides for a reliable and quick method to provide for complementary and reproducible subsets of a genome that can be sequenced. In certain embodiments, the primers used in the complexity reduction contain one or more thioate linkages to increase their selectivity and/or performance.

In certain alternative embodiments, complexity reduction comprises the CHIP method. Other suitable methods for complexity reduction are Chromatine Immuno Precipitation (ChiP). This means that nuclear DNA is isolated, whilst proteins such as transcription factors are linked to the DNA. With Chip first an antibody is used against the protein, resulting in Ab-protein-DNA complex. By purifying this complex and precipitating it, DNA to which this protein binds is selected. Subsequently, the DNA can be used for library construction and sequencing. I.e., this is a method to perform a complexity reduction in a non-random fashion directed to specific functional areas; in the present example specific transciption factors. Alternative embodiments may use the design of PCR primers directed against conserved motifs such as SSRs, NBS regions (nucleotide biding regions), promoter/enhancer sequences, telomer consensus sequences, MADS box genes, ATP-ase gene families and other gene families.

In step (e), first, second and further sequencing libraries are generated for each subset of amplified adaptor-ligated restriction fragments. The libraries are typically generated by fragmentation of the amplified adaptor-ligated restriction fragments. Fragmentation can be achieved by physical techniques, i.e. shearing, sonication or other random fragmentation methods. In step (f), at least part, but preferably the entire, nucleotides sequence of at least part of, but preferably of all the fragments contained in the libraries is determined.

The sequencing may in principle be conducted by any means known in the art, such as the dideoxy chain termination method. It is however preferred that the sequencing is performed using high-throughput sequencing methods, such as the methods disclosed in WO 03/004690, WO 03/054142, WO 2004/069849, WO 2004/070005, WO 2004/070007, and WO 2005/003375 (all in the name of 454 Life Sciences), by Seo et al. (2004) Proc. Natl. Acad. Sci. USA 101:5488-93, and technologies of Helios, Solexa, US Genomics. It is most preferred that sequencing is performed using the apparatus and/or method disclosed in WO 03/004690, WO 03/054142, WO 2004/069849, WO 2004/070005, WO 2004/070007, and WO 2005/003375 (all in the name of 454 Life Sciences). The technology described allows sequencing of 40 million bases in a single run and is 100 times faster and cheaper than competing technology. The sequencing technology roughly consists of 5 steps: 1) fragmentation of DNA and ligation of specific adaptor to create a library of single-stranded DNA (ssDNA); 2) annealing of ssDNA to beads, emulsification of the beads in water-in-oil microreactors and performing emulsion PCR to amplify the individual ssDNA molecules on beads; 3) selection of /enrichment for beads containing amplified ssDNA molecules on their surface 4) deposition of DNA carrying beads in a PicoTiterPlate®; and 5) simultaneous sequencing in 100,000 wells by generation of a pyrophosphate light signal. The method will be explained in more detail below.

In a preferred embodiment, the sequencing comprises the steps of:
(a) annealing adapted fragments to beads, each bead being annealed with a single adapted fragment;
(b) emulsifying the beads in water-in-oil microreactors, each water-in-oil microreactor comprising a single bead;
(c) loading the beads in wells, each well comprising a single bead; and generating a pyrophosphate signal.

In the first step (a), sequencing adaptors are ligated to fragments within the combination library. Said sequencing adaptor includes at least a "key" region for annealing to a bead, a sequencing primer region and a PCR primer region. Thus, adapted fragments are obtained.

In a first step, adapted fragments are annealed to beads, each bead annealing with a single adapted fragment. To the pool of adapted fragments, beads are added in excess as to ensure annealing of one single adapted fragment per bead for the majority of the beads (Poisson distribution).

In a next step, the beads are emulsified in water-in-oil microreactors, each water-in-oil microreactor comprising a single bead. PCR reagents are present in the water-in-oil microreactors allowing a PCR reaction to take place within the microreactors. Subsequently, the microreactors are broken, and the beads comprising DNA (DNA positive beads) are enriched.

In a following step, the beads are loaded in wells, each well comprising a single bead. The wells are preferably part of a PicoTiter™Plate allowing for simultaneous sequencing of a large amount of fragments.

After addition of enzyme-carrying beads, the sequence of the fragments is determined using pyrosequencing. In successive steps, the PicoTiter™Plate and the beads as well as the enzyme beads therein are subjected to different deoxyribonucleotides in the presence of conventional sequencing reagents, and upon incorporation of a deoxyribonucleotide a light signal is generated which is recorded. Incorporation of the correct nucleotide will generate a pyrosequencing signal which can be detected.

Pyrosequencing itself is known in the art and described inter alia on www.biotagebio.com; www.pyrosequencing.com / section technology. The technology is further applied in e.g. WO 03/004690, WO 03/054142, WO 2004/069849, WO 2004/070005, WO 2004/070007, and WO 2005/003375 (all in the name of 454 Life Sciences).

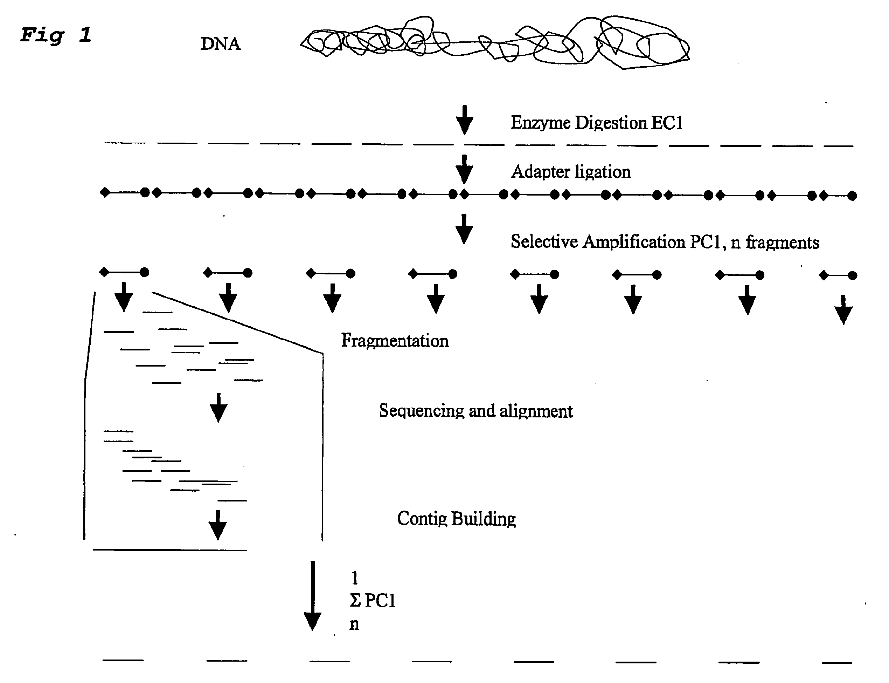

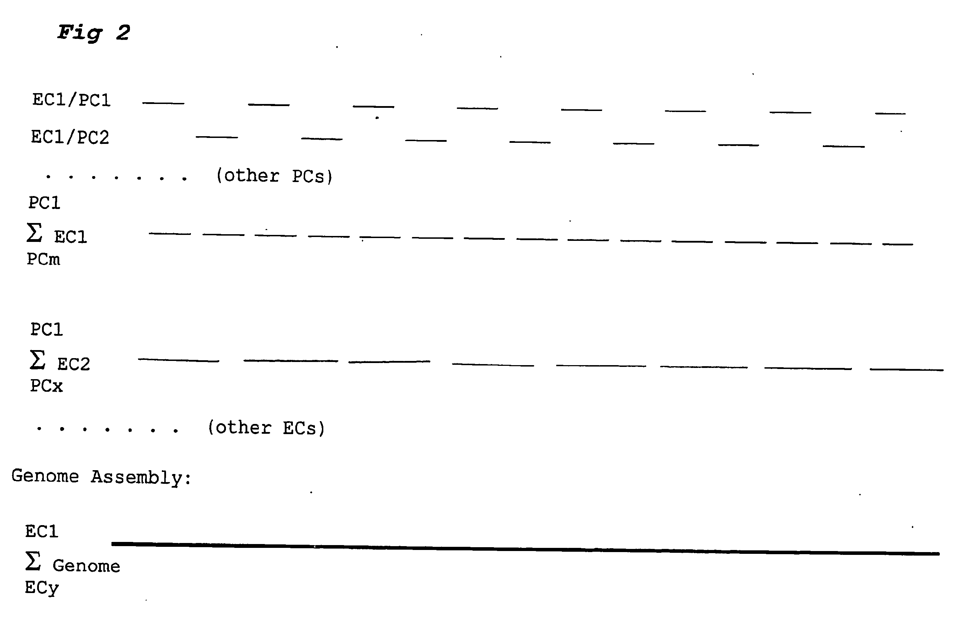

In step (g) of the method of the invention, the determined sequences of the fragments of the first, second and/or further libraries are aligned. The alignment provides contigs of the fragments in the subsets of the amplified adaptor-ligated restriction fragments. In this way for each amplified adaptor-ligated restriction fragment, a contig is generated from sequenced fragments, i.e. the contig of one amplified adaptor-ligated restriction fragment, is build up from the alignment of the sequence of the various fragments obtained from the fragmenting in step (e). By building contigs from sequences representing dispersed restriction fragments of a small portion of the genome, problems with contig building resulting from abundant repeat sequences are greatly diminished leading to a higher quality draft genome sequence which contains less errors due to false-joining of repeated sequences. In addition, the assembly process will be computationally less complex and therefore faster to perform. By aligning the sequences in the different libraries, contigs for each restriction fragment of the set of restriction fragments can be build for each primer combination. This results in a set of contigs, each corresponding to a particular restriction fragment. As a result, each restriction fragment obtained from the restriction of the genome with the at least one restriction endonuclease has now a determined (contig) sequence. The method of the invention is illustrated in Fig 1 and 2.

Methods of alignment of sequences for comparison purposes are well known in the art. Various programs and alignment algorithms are described in: Smith and Waterman (1981) Adv. Appl. Math. 2:482; Needleman and Wunsch (1970) J. Mol. Biol. 48:443; Pearson and Lipman (1988) Proc. Natl. Acad. Sci. USA 85:2444; Higgins and Sharp (1988) Gene 73:237-244; Higgins and Sharp (1989) CABIOS 5:151-153; Corpet et al. (1988) Nucl. Acids Res. 16:10881-90; Huang et al. (1992) Computer Appl. in the Biosci. 8:155-65; and Pearson et al. (1994) Meth. Mol. Biol. 24:307-31, which are herein incorporated by reference. Altschul et al. (1994) Nature Genet. 6:119-29 (herein incorporated by reference) present a detailed consideration of sequence alignment methods and homology calculations.

The NCBI Basic Local Alignment Search Tool (BLAST) (Altschul et al., 1990) is available from several sources, including the National Center for Biological Information (NCBI, Bethesda, Md.) and on the Internet, for use in connection with the sequence analysis programs blastp, blastn, blastx, tblastn and tblastx. It can be accessed at <http://www.ncbi.nlm.nih.gov/BLAST/>. A description of how to determine sequence identity using this program is available at <http://www.ncbi.nlm.nih.gov/BLAST/blast-help.html>. A further application can be in microsatellite mining (see Varshney et al. (2005) Trends in Biotechn. 23(1):48-55.

Typically, the alignment is performed on sequence data that have been trimmed for the adaptors/primer and/or identifiers but with reconstructed restriction enzyme recognition sequences, i.e. using only the sequence data from the fragments that originate from the nucleic acid sample. Typically, the sequence data obtained are used for identifying the origin of the fragment (i.e. from which sample), the sequences derived from the adaptor and/or identifier are removed from the data and alignment is performed on this trimmed set.

In step (h), the whole procedure is repeated at least once with one or more different restriction endonucleases, i.e. a restriction endonuclease that, preferably, contains a different recognition site than the first endonuclease, to provide for a second or even further set of restriction fragments that are subsequently adaptor ligated, selectively amplified using primer combinations with a selected sequence that is independently selected, i.e. bears no relationship with the selected sequence,(either in number or in type of nucleotides) with the ones that have been used for the same purpose with the first restriction endonuclease. For example, the first subset can be obtained by restriction with MseI/PstI and selectively amplified using a selective primer for the MseI-remains of the recognition site and that carries 2 selective nucleotides at its 3' end. The second subset can be obtained by EcoRI/HindIII digestion and selective amplification with a selective primer for the EcoRI-remains of the recognition site with 1 selective nucleotide.

Thus a second (and/or further) set of contigs for all restriction fragments can be obtained in this way, in a similar manner as disclosed herein before. This is necessary, as for a given restriction endonuclease, the fractions of the genome that are being sequenced are complementary, they do not overlap. The contigs obtained with different enzyme combination do overlap and hence allow the generation of a contig therefrom and hence allow the formation of a (draft) genome sequence.

In step (i) of the method, the contigs obtained from the previous steps of the method for each fragment are aligned to form a sequence of the genome.

In certain embodiments, the contig building of either the restriction fragments or of the genome sequence can be aided by the use of nucleotide sequences of the genome that are derived from other sources, including, but not limited to BAC-end sequences, BAC shotgun sequences, EST sequences or whole genome shotgun sequences.

The method of the present invention is independent of the source of DNA, i.e. applicable to all organisms as it does not require any previous sequence information. By appropriate selection of enzymes, adaptors, primers and number of selective nucleotides scalable technology is presented that is applicable to genomes of all sizes and complexities. Furthermore the genome fractions that are obtained with the different primer combinations and/or with the selective primers that differ from each other in the specific selective nucleotide sequence at the 3'-end, are complementary. This means that when, for any given number of selective nucleotides, all permutations are being used (1 selective nucleotide equals 4 variants (A, C, T,G), 2 selective nucleotides 16, 3 selective nucleotides 64 variations and so on), the combined restriction fragments constitute the restricted genome.

### Brief description of the drawings

**Figure 1****:** Starting from genomic DNA, a digestion with a combination of restriction endonucleases (Enzyme Combination 1, EC1) is performed, resulting in a set of restriction fragments. To the restriction fragments, adapters are ligated after which the adapter-ligated restriction fragments are amplified with a first selective primer combination (PC1) to result in n fragments. Each fragment is fragmented for high throughput sequencing and subjected to sequencing and alignment to generate contigs of the restriction fragments. In this way, the sequence of all or most of the amplified adapter-ligated restriction fragments n is determined for one primer combination.

**Figure 2****:**For each possible primer combination for a given enzyme combination (EC1), the steps of fragmentation of the amplified adapter-ligated restriction fragments, sequencing, alignment and contig building are repeated. This means that when, for instance, the selective amplification is performed with primers that each carry a selective nucleotide at their 3' end (i.e. +1/+1 primers), 16 primer combinations (PC1...PCm) cover all permutations and with 16 primer combinations all adapter-ligated restriction fragments have been amplified and subsequently sequenced. From the contigs generated with EC1, i.e. from EC1/PC1 ...EC1/PCm, an assembly will cover a large part of the genome, but needs to be anchored in order to provide a genome sequence. For this purpose, a second enzyme combination (and, if necessary a third and a fourth etc.) is used. The steps of fig 1 and 2 are repeated with enzyme combination 2 (EC2), i.e. restriction, adapter-ligation etc. The selective amplification is performed with a set of selective primers that that typically may be different (sequence and selectivity) from the primers used with EC1. The adapter-ligated restriction fragments are again amplified with all possible permutations of a set of selective primers yielding different and complementary subsets. Fragmentation of each subset of selectively amplified adapter-ligated restriction fragments, and subsequent high throughput sequencing, contig building etc. leads to a second assembly, again covering a large part of the genome. From these two assemblies (and the optional third, fourth etc enzyme combination), which overlap each other for large area's, the draft sequence of the genome that is investigated, is generated.

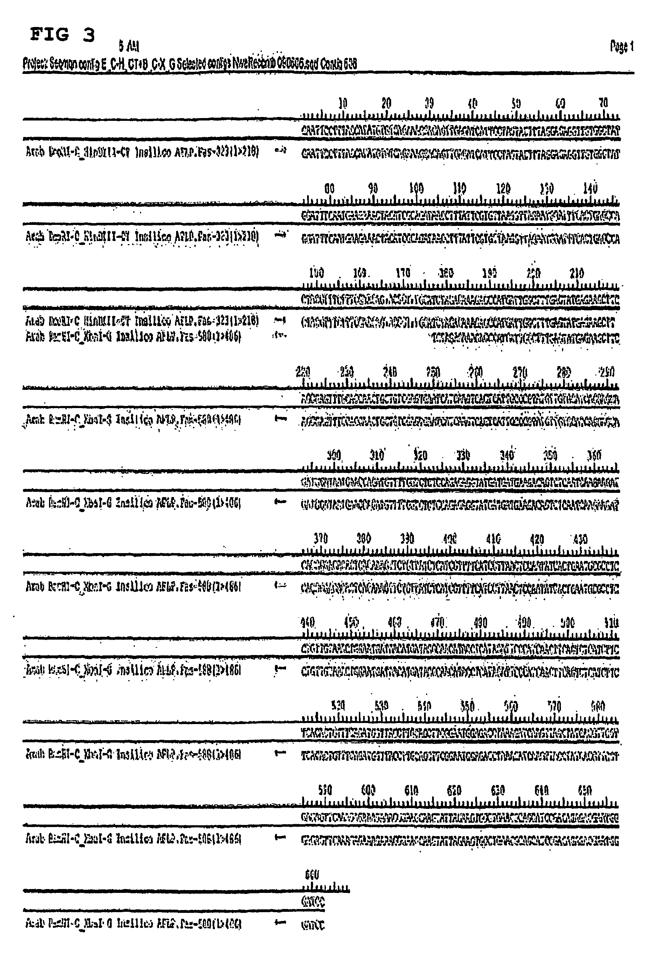

**Figure 3****.** *In silico* predicted 662 bp sequence of contig 606, containing overlapping EcoRI/HindIII +C/+CT and BamHI/XbaI +C+G restriction fragments.

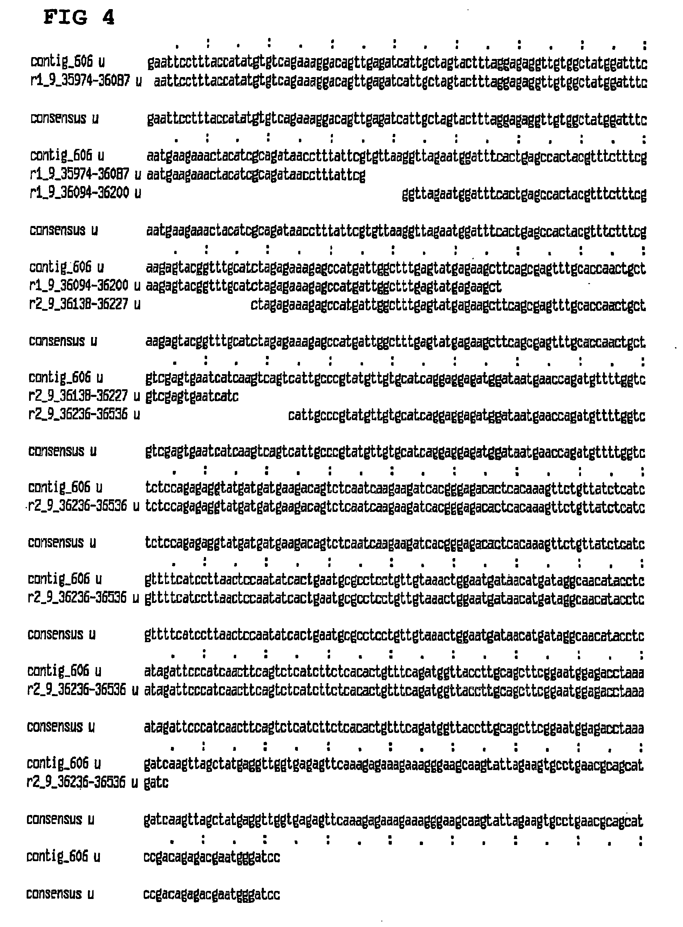

**Figure 4****.** Observed sequence contigs of *in silico* predicted contig 606 based on sequencing AFLP fragment libraries EcoRI/HindIII +C/+CT (r1_9_35974-36087) and BamHI/XbaI +C/+G (r2_9_36138-36200). Note that the 42 bp overlap between (r1_9_35974-36087) and (r2_9_36138-36200) is fully covered by the sequences obtained from both fragment libraries.

The invention can be illustrated by means of the following examples that are not intended to limit the invention in any way, but merely serve as an illustration.

### Whole genome sequencing using Long-range PCR.

Step 1: DNA is restricted using two 6 cutters A and B for instance EcoRI and HindIII). This generates three types of restriction fragments: A-A (25%), B-B (25%) and A-B (50%) with an average length of about 3-4 kb, depending on the GC-content of the genome of interest, and the selection of the restriction endonucleases. After ligation of adaptors, long-range PCR is performed with +X/+Y primers (i.e. one of the primers contains X selective nucleotides and the other Y), to 1 Mb sequence per primer combination. In the case that X=2 and Y=3, repeat this for all 1024 primer combinations. In the case that X=1 and Y=2, repeat this for all 64 primer combinations.
A: Maize 2700 Mbp genome size: type A-B fragments =1350 Mbp. By a +2/+3 selective amplification (1024 primer combinations) the amplification product of each primer combinations contains on average 1350/1024=1.32 Mbp sequence. With an average length per A-B fragment of about 3000 bp, this yield 1320000/3000= 440 A-B fragments.
B: Arabidopsis 130 Mbp: type A-B fragments 65Mbp. With X=1 and Y=2, every primer combination (PC) contains about 1 Mbp sequence. With an average length of 3000 bp per fragment this is 1000000/3000=330 A-B fragments.

Step 2: construction of libraries by shearing of each set of amplified adaptor-ligated restriction fragments and sequencing using the emulsion PCR in combination with pyrosequencing of 454 Technologies as described herein elsewhere, for each primer combination (1024 or 64 times). The sequencing using this technology provides for 40Mbp sequence data per library, meaning that every library is 40-fold redundantly sequenced. By variation of the number of nucleotides, a different amount of A-B fragments are amplified and a different redundancy will be achieved. This can be determined in practice.

Step 3: assembly of the sequences per sequence library (per PC)

Assembly is performed to generate contigs of all A-B fragments that have been amplified per PC. This leads to about 300 to 500 contigs per PC of which the average length will correspond to the average length of the A-B fragment. The sequencing of all PCs results in a number of contigs that varies from several ten thousand up to several hundred thousands (Arabidopsis 21000, Maize 450000).

Step 4: repeat steps 1-3 for at least one other enzyme combination (EC), for instance A-C. This is obligatory because all PCs from EC A-B only provide complementary contigs and not overlapping contigs, which cover only 50% of the genome. The coverage of the genome can be enhanced by also processing all A-A and B-B fragments. By using additional ECs, overlap is achieved between the contigs of AB and of AC and the genome coverage increases.

Step 5: assemble all contigs of A-B (optionally also A-A, B-B) and A-C primer combinations to a (draft) genome sequence.

One of the advantages of this method resides in the fact that one of the problems of genome assembly and the chance on the formation of wrong contig due to the manifold presence of repeat sequences is being minimized by the formation of small dispersed (.i.e., non-adjacent) contigs of 1-10 kb within a 1-5 Mbp fraction of the genome instead of the whole genome. Contigs with lengths that are much larger can be labelled in an early stage as being the product of false joining and discarded. A further advantage is that assembly is computationally less complex because at the initial assembly (step 3), less sequences are involved than when the entire genome sequence is to be assembled in one step. A further advantage is that the selective amplification process renders the entire process scalable to any size genome and it is universally applicable.

### Whole genome sequencing using one rare and one frequent cutter.

Step 1: as above, with a 6-cutter (EcoRI) and a 4-cutter (MseI). The average fragment length is about 250 bp. The A-B fragments represent about 8-15% of the genome. Compared with restriction enzyme digestion using two 6-cutter restriction enzymes, on average about 1 selective nucleotide less is needed to come to an amount of sequence complexity per PC of about 1-5 Mbp.
**A:** Maize 2700 Mbp genome size: type A-B fragments =270 Mbp, (10%) by a +2/+2 selective amplification (256 primer combinations) contains the amplification product of each primer combinations on average 270/256=1.05 Mbp sequence. With an average length per A-B fragment of about 250 bp, this yield 1050000/250= 4200 A-B fragments/contigs.
**B:** Arabidopsis 130 Mbp: type A-B fragments 13 Mbp (10%). With X=1 and Y=1, every primer combination (PC) contains about 1 Mbp sequence. With an average length of 250 bp per fragment this is 1000000/3000=4000 A-B fragments.

Step 2: as above. To avoid bias of too short fragments, a size selection can be used to remove fragments below 100-150 bp.

Step 3: Assembly of the sequences per sequence library (per PC)

Assembly is performed to generate contigs of all A-B fragments that have been amplified per PC. This leads to several thousands of contigs per PC of which the average length will correspond to the length of the A-B fragment (250bp). The sequencing of all PCs results in a number of contigs that varies from several ten thousand up to about a million (Arabidopsis 64000, Maize 1000000).

Step 4: repeat steps 1-3 with a variety of ECs (A-C, B-C, C-C, C-D, A-D etc). This is necessary as the PCs of enzyme combination A-B do not cover more than 8-15 % of the genome and, as above, the contigs of the PCs do not overlap.

Step 5: as above.

### Whole genome sequencing using one restriction endonuclease.

Step 1: Digest the DNA with one restriction endonuclease A (EcoRI for example). Restriction fragments of about 3-4 kb, depending on GC content and choice of enzyme. Ligate mix to adaptor and perform Long range PCR (see above) with selective primers that reduce the amount of sequence per PC to about 1 Mb. In the case of X=2 and Y=3 repeat for all 1024 PCs. For (X,Y)=(+1/+2) repeat for all 64 PCs.
**A:** Maize 2700 Mbp genome size: type A-A fragments =2700 Mbp, by a +2/+3 selective amplification (1024 primer combinations) contains the amplification product of each primer combinations on average 2700/1024=2.64 Mb sequence. With an average length per A-A fragment of about 3000 bp, this yield 2640000/300= 880 A-A fragments/contigs.
**B:** Arabidopsis 130 Mbp: type A-A fragments 130 Mb. With X=1 and Y=2, every primer combination (PC) contains about 2 Mb sequence. With an average length of 3000 bp per fragment this is 2000000/3000=660 A-A fragments.

Step 2: construction of libraries by shearing of each set of amplified adaptor-ligated restriction fragments and sequencing using the emulsion PCR in combination with pyrosequencing of 454 Technologies as described herein elsewhere, for each primer combination (1024 or 64 times). The sequencing using this technology provides for 40 Mbp sequence data per library, meaning that every library is 20-fold redundantly sequenced.

Step 3: Assembly of the sequences per sequence library (per PC)

Assembly is performed to generate contigs of all A-A fragments that have been amplified per PC. This leads, theoretically, to 600-900 of contigs per PC of which the average length will correspond to the length of the A-A fragment (3000bp). The sequencing of all PCs results in a number of contigs that varies from several tens up to about hundreds of thousands (Arabidopsis 42000, Maize 900000).

Step 4: repeat steps 1-3 with at least one other ECs (B-B). This is necessary as the PCs of enzyme combination A-B do not cover more than 8-15 % of the genome and, as above, the contigs of the PCs do not overlap.

Step 5: as above.

### Example 1

This example describes the ability to use high throughput sequencing of AFLP fragments derived from 2 restriction enzyme combinations to determine the genome sequence of a complex plant genome.

The following steps were taken in this example:

*A)in silico* prediction of AFLP restriction fragments of the Arabidopsis genome sequence (Genbank), using the software tool RECOMB, described in WO0044937 (Keygene N.V).

The entire genome sequence of Arabidopsis genome (ecotype Colombia) was downloaded from Genbank. *In silico* AFLP +1/+1 fragments for the restriction enzyme combination BamHI/XbaI using +C and +G selective nucleotides, respectively, were predicted using RECOMB. Similarly, AFLP +1/+2 fragments for the restriction enzyme combination EcoRI/HindIII using selective nucleotides +C and +CT were predicted. The collection of AFLP fragments derived from the two *in silico* digests resulted in various (of approximately 14) overlapping AFLP fragment sequences between the enzyme combinations BamHI/XbaI and EcoRI/HindIII. One of the overlapping restriction fragments forms a contig denoted contig "606", which has a total length of 662 bp. The sequence of this contig is shown in Figure 3.

The predicted EcoRI/HindIII AFLP +C/+CT fragment in this contig is 218 bp in length, accounting for 32,9 % of the total contig length of 606 bp. The predicted BamHI/XbaI AFLP +C/+G fragment is 486 bp long, equaling 73,4% of the total contig length. Both fragments overlap by 42 basepairs as depicted in Figure 3.

B) AFLP template preparation and amplification

Genomic DNA of the *Arabidopsis* ecotype Colombia and AFLP templates for the restriction enzyme combinations EcoRI/HindIII and BamHI/XbaI were prepared based on the protocols described by Zabeau & Vos, 1993: Selective restriction fragment amplification; a general method for DNA fingerprinting. EP 0534858-A1, B1; US patent 6045994) and Vos et al (Vos,P., Hogers,R., Bleeker,M., Reijans,M., van de Lee,T., Hornes,M., Frijters,A., Pot,J., Peleman,J., Kuiper,M. et al. (1995) AFLP: a new technique for DNA fingerprinting. Nucl. Acids Res., 21, 4407-4414).

The following adaptor sequences (5'-3') were used:
BamHI:
   91M35: CTCGTAGACTGCGTACC [SEQ ID 1]
   93U01: GATCGGTACGCAGTC [SEQ ID 2]
XbaI:
   90K02: CTCGTAGACTGCGTACA [SEQ ID 3]
   92A16: CTAGTGTACGCAGTCT [SEQ ID 4]
EcoRI:
   91M35: CTCGTAGACTGCGTACC[SEQ ID 5]
   91M36: AATTGGTACGCAGTCTAC[SEQ ID 6]
HindIII:
   91M35: CTCGTAGACTGCGTACC [SEQ ID 7]
   91M37: AGCTGGTACGCAGTCTAC [SEQ ID 8]

Selective (+1/+1) amplifications (E/H and B/X) were carried out using the following phosphorothioate primers (5'-3'):

BamHI+C-thio: 96R22thio: GACTGCGTACCGATCsCsC [SEQ ID 9]

XbaI+G: 96X03thio: GACTGCGTACACTAGsAsG [SEQ ID 10]

EcoRI+C-thio: 93T14thio: GACTGCGTACCAATTsCsC [SEQ ID 11]

HindIII+C-thio: 95H18thio GACTGCGTACCAGCTTsCsT [SEQ ID 12]

with an "s" is denoting the position of phosphorothioate bonds on the oligonucleotides.

AFLP reactions mixtures had the following composition:
5 ul 1/10 in MQ diluted AFLP template
10 ul 5x herculase II PCR buffer
0.5 ul dNTP's (20mM)
1.5 ul AFLP primer 1 (50ng/ul)
1.5 ul AFLP primer 2 (50ng/ul)
1 ul Herculase® II Fusion DNA-polymerase
30.5 ul MQ

PCR cycling conditions were as follows:
Initial denaturation 94°C 2 min
Denaturation 94°C 10sec
Annealing 56°C 30sec 10 cycli
Elongation 68°C 2 min
Denaturation 94°C 15sec
Annealing 56°C 30sec 20 cycli
Elongation 68°C 2 min*
   *Touch up: 20 sec per cycle

Following AFLP amplification, reactions products were purified using Qiagen columns following the manufacturers protocols

C) 454 sequence library preparation.

Two 454 sequence libraries were prepared using purified BamHI/XbaI AFLP fragments and EcoRI/HindIII AFLP fragments as starting DNA respectively, as described by Margulies and co-workers, starting with nebulization (fragmentation) of the purified AFLP reaction products. A single 454 sequence run was performed using on the GS20 sequencing instrument (Roche Molecular Diagnostics), applying each of the fragment libraries of the two AFLP enzyme combinations to one half of a GS20 PicoTiterPlate.

D) Data processing

After completion of the sequence run, raw data were processed using the RUNASSEMBLY software of the GS20. Data resulting from the EcoRI/HindIII and BamHI/XbaI AFLP fragment libraries were processed separately and in combination, generating contigs of overlapping sequence reads.

Next, contigs resulting from RUNASSEMBLY were mapped against the reference genome (contig 606 predicted in step a) above using RUNMAPPING, in order to determine to which extent the *in silico* predicted BamHI/XbaI +C/+G and EcoRI/HindIII +C/+CT AFLP fragments contained in contig 606 were sequenced. Coverage percentages obtained from the respective libraries are shown in the table below.

**table : Summary of sequence coverage of Arabidopsis contig 606**

| | Expected % | Observed number of contigs within contig 606 | Observed % sequence coverage within contig 606 | Overlap length (bp) | Coverage overlap (bp) | Coverage overlap (%) |
|---|---|---|---|---|---|---|
| EcoRI/ HindIII | 32,9 | 3 | 35,2 | 42 | 42 | 100 |
| BamHI/ XbaI | 71,4 | 2 | 59,0 | 42 | 42 | 100 |
| EcoRI/ HindIII + BamHI/ XbaI | 100 | 4 | 84,8 | 42 | 42 | 100 |

The resulting sequence contigs are shown in **Figure 4****.**

These results demonstrate the feasibility to determine the genome sequence of complex plant genomes by digesting total genomic DNA with multiple AFLP restriction enzyme combinations, followed by contig assembly per fragment library, and subsequent assembly of the contigs into the plant genome sequence.

### SEQUENCE LISTING

<110> Keygene NV
<120> Improved strategies for sequencing complex genomes using High Throughput sequencing technologies.
<130> P27925PC00
<150> US60/693,053
   <151> 2005-06-23
<150> US60/714,897
   <151> 2005-09-08
<150> EP06075104.7
   <151> 2006-01-16
<150> US60/759,034
   <151> 2006-01-17
<160> 12
<170> PatentIn version 3.3
<210> 1
   <211> 17
   <212> DNA
   <213> Artificial
<220>
   <223> adaptor
<400> 1
   ctcgtagact gcgtacc 17
<210> 2
   <211> 15
   <212> DNA
   <213> Artificial
<220>
   <223> adaptor
<400> 2
   gatcggtacg cagtc 15
<210> 3
   <211> 17
   <212> DNA
   <213> Artificial
<220>
   <223> adaptor
<400> 3
   ctcgtagact gcgtaca 17
<210> 4
   <211> 16
   <212> DNA
   <213> Artificial
<220>
   <223> adaptor
<400> 4
   ctagtgtacg cagtct 16
<210> 5
   <211> 17
   <212> DNA
   <213> Artificial
<220>
   <223> adaptor
<400> 5
   ctcgtagact gcgtacc 17
<210> 6
   <211> 18
   <212> DNA
   <213> Artificial
<220>
   <223> adaptor
<400> 6
   aattggtacg cagtctac 18
<210> 7
   <211> 17
   <212> DNA
   <213> Artificial
<220>
   <223> adaptor
<400> 7
   ctcgtagact gcgtacc 17
<210> 8
   <211> 18
   <212> DNA
   <213> Artificial
<220>
   <223> adaptor
<400> 8
   agctggtacg cagtctac 18
<210> 9
   <211> 17
   <212> DNA
   <213> Artificial
<220>
   <223> primer
<220>
   <221> modified_base
   <222> (16)..(17)
   <223> phosphorothioate bonds
<400> 9
   gactgcgtac cgatccc 17
<210> 10
   <211> 17
   <212> DNA
   <213> Artificial
<220>
   <223> primer
<220>
   <221> modified base
   <222> (16)..(17)
   <223> phosphorothioate bonds
<400> 10
   gactgcgtac cgatccc 17
<210> 11
   <211> 17
   <212> DNA
   <213> Artificial
<220>
   <223> primer
<220>
   <221> modified base
   <222> (16)..(17)
   <223> phosphorothioate bonds
<400> 11
   gactgcgtac cgatccc 17
<210> 12
   <211> 19
   <212> DNA
   <213> Artificial
<220>
   <223> primer
<220>
   <221> modified_base
   <222> (16)..(17)
   <223> phosphorothioate bonds
<400> 12
   gactgcgtac cgatcscsc 19

## Claims

1. A method for determining a genome sequence comprising the steps of:
(a) providing a first subset of the genome by digesting the genome with at least one first restriction endonuclease to provide restriction fragments;
(b) ligating at least one adaptor to the restriction fragments of the first subset to provide a first set of adaptor-ligated restriction fragments:
(c) selectively or non-selectively amplifying the first set of adaptor-ligated restriction fragments using a first primer combination wherein at least a first primer contains a section that is complementary to the adaptor and to part of the recognition sequence of the restriction endonuclease and that further contains a first selected sequence at the 3' end of the primer sequence, wherein the first selected sequence comprises 0-10 selective nucleotides, to provide a first subset of amplified adaptor-ligated restriction fragments;
(d) repeating step (c) with at least a second and/or further primer combinations wherein the primer contains a different second and/or further selected sequence at its 3'end that contains the same number of selective nucleotides, to provide for second and/or further subsets of amplified adaptor-ligated restriction fragments;
(e) fragmenting each of the first, second and/or further subsets of amplified adaptor-ligated restriction fragments to generate first, second and/or further sequencing libraries;
(f) determine at least part of the nucleotide sequence of at least part of the fragments contained in each of the first, second and/or further libraries;
(g) aligning the sequence of the fragments in each of the first, second and/or further libraries to generate contigs of the amplified adaptor-ligated restriction fragments derived representing dispersed fractions of the genome;
(h) repeating steps (a)-(g) for at least one second and /or further restriction endonucleases;
(i) aligning the contigs obtained in step (g) and (h) for each of the second and /or further restriction endonucleases to provide for a sequence of the genome.

2. Method according to claim 1, wherein at least one of the first, second and/or further restriction endonucleases is a rare cutter.

3. Method according to claim 1 or 2, wherein at least one of the first, second and/or further restriction endonuclease is a frequent cutter.

4. Method according to claim 1, 2 or 3, wherein at least two rare cutters are used.

5. Method according to claims 1-4, wherein one rare cutter and one frequent cutter are used.

6. Method according to claims 1-5, wherein the amplification method is PCR.

7. Method according to claims 1-6 wherein the selected sequence at the 3' end of the primer contains 1-8 selected nucleotides.

8. Method according to claims 1-7, wherein the first, second and further selected sequence have the same number of nucleotides but differ in nucleotide sequence from each other in the selective sequence located at the 3'-end of the primer.

9. Method according to claims 1-8, wherein sequencing is performed by Sanger dideoxy sequencing.

10. Method according to claims 1-8, wherein sequencing is performed on a solid support such as a bead.

11. Method according to claims 1-8, wherein the sequencing is based on High Throughput Sequencing.

12. Method according to claims 1-8, wherein the sequencing is based on Sequencing-by-Synthesis.

13. Method according to claims 1-8, wherein the sequencing is based on Pyrosequencing.

14. Method according to claims 1-8, wherein sequencing comprises the steps of:
(f1) ligating sequencing-adaptors to the fragments;
(f2) annealing sequencing-adaptor-ligated fragments to beads, each bead annealing with a single fragment,
(f3) emulsifying the beads in water-in-oil micro reactors, each water-in-oil micro reactor comprising a single bead;
(f4) performing emulation PCR to amplify adaptor-ligated fragments on the surface of beads
(f5) selecting / enriching beads containing amplified adaptor-ligated fragments
(f6) loading the beads in wells, each well comprising a single bead; and
(f7) generating a pyrophosphate signal.

15. Methods according to any of the preceding claims, wherein contig building is further aided by the use of nucleotide sequences derived from other sources, including, but not limited to BAC-end sequences, BAC shotgun sequences, EST sequences or whole genome shotgun sequences.

16. Method according to any of the previous claims, wherein the method for reducing the complexity of the mixture is based on indexing linkers, Chromatine Immuno Precipitation (CHIP) or PCR primers directed against conserved motifs.

## Patentansprüche

1. Verfahren zum Bestimmen einer Genomsequenz, umfassend die Schritte:
(a) Bereitstellen einer ersten Teilmenge des Genoms durch Verdauen des Genoms mit mindestens einer ersten Restriktionsendonuclease, um Restriktionsfragmente bereitzustellen;
(b) Ligieren von mindestens einem Adapter an die Restriktionsfragmente der ersten Teilmenge, um eine erste Menge Adapter-ligierter Restriktionsfragmente bereitzustellen;
(c) selektives oder nicht selektives Amplifizieren der ersten Menge Adapter-ligierter Restriktionsfragmente unter Verwendung einer ersten Primerkombination, wobei mindestens ein erster Primer einen Abschnitt enthält, der zu dem Adapter und zu einem Teil der Erkennungssequenz der Restriktionsendonuclease komplementär ist und der ferner eine erste ausgewählte Sequenz am 3'-Ende der Primersequenz enthält, wobei die erste ausgewählte Sequenz 0 - 10 selektive Nucleotide umfasst, um eine erste Teilmenge amplifizierter Adapter-ligierter Restriktionsfragmente bereitzustellen;
(d) Wiederholen von Schritt (c) mit mindestens einer zweiten und/oder weiteren Primer-Kombinationen, wobei der Primer eine andere zweite und/oder weitere ausgewählte Sequenz am 3'-Ende enthält, welche die gleiche Anzahl selektiver Nucleotide enthält, um eine zweite und/oder weitere Teilmengen amplifizierter Adapter-ligierter Restriktionsfragmente bereitzustellen;
(e) jeweils Fragmentieren der ersten, zweiten und/oder weiteren Teilmengen amplifizierter Adapter-ligierter Restriktionsfragmente, um erste, zweite und/oder weitere Sequenzierbanken zu erzeugen;
(f) Bestimmen von mindestens einem Teil der Nucleotidsequenz von mindestens einem Teil der in jeweils der ersten, zweiten und/oder weiteren Banken enthaltenen Fragmente;
(g) Abgleichen der Sequenz der Fragmente in jeweils der ersten, zweiten und/oder weiteren Banken, um Contigs der erhaltenen amplifizierten Adapter-ligierten Restriktionsfragmente zu erzeugen, die verstreute Bruchteile des Genoms darstellen;
(h) Wiederholen der Schritte (a) - (g) mit mindestens einer zweiten und/oder weiteren Restriktionsendonucleasen;
(i) Abgleichen der in Schritt (g) und (h) erhaltenen Contigs für jeweils die zweite und/oder weitere Restriktionsendonucleasen, um eine Sequenz des Genoms bereitzustellen.

2. Verfahren gemäß Anspruch 1, wobei es sich bei mindestens einer der ersten, zweiten und/oder weiteren Restriktionsendonucleasen um eine selten schneidende handelt.

3. Verfahren gemäß Anspruch 1 oder 2, wobei es sich bei mindestens einer der ersten, zweiten und/oder weiteren Restriktionsendonucleasen um eine häufig schneidende handelt.

4. Verfahren gemäß den Ansprüchen 1, 2 oder 3, wobei mindestens zwei selten schneidende Restriktionsendonucleasen verwendet werden.

5. Verfahren gemäß den Ansprüchen 1 - 4, wobei eine selten schneidende und eine häufig schneidende Restriktionsendonuclease verwendet werden.

6. Verfahren gemäß den Ansprüchen 1 - 5, wobei es sich bei dem Amplifikationsverfahren um PCR handelt.

7. Verfahren gemäß den Ansprüchen 1 - 6, wobei die ausgewählte Sequenz am 3'-Ende des Primers 1 - 8 ausgewählte Nucleotide enthält.

8. Verfahren gemäß den Ansprüchen 1 - 7, wobei die erste, zweite und weitere ausgewählte Sequenz die gleiche Anzahl Nucleotide aufweisen, sich aber in der selektiven, am 3'-Ende des Primers liegenden Sequenz in der Nucleotidsequenz voneinander unterscheiden.

9. Verfahren gemäß den Ansprüchen 1 - 8, wobei die Sequenzierung durch Sanger-Dideoxy-Sequenzierung durchgeführt wird.

10. Verfahren gemäß den Ansprüchen 1 - 8, wobei die Sequenzierung auf einer festen Unterlage, wie zum Beispiel einem Kügelchen, durchgeführt wird.

11. Verfahren gemäß den Ansprüchen 1 - 8, wobei die Sequenzierung auf High-Throughput-Sequenzierung basiert.

12. Verfahren gemäß den Ansprüchen 1 - 8, wobei die Sequenzierung auf Sequenzierung-durch-Synthese basiert.

13. Verfahren gemäß den Ansprüchen 1 - 8, wobei die Sequenzierung auf Pyrosequenzierung basiert.

14. Verfahren gemäß den Ansprüchen 1 - 8, wobei die Sequenzierung die Schritte umfasst:
(f1) Ligieren von Sequenzieradaptoren an die Fragmente;
(f2) Anlagern von Sequenzieradapter-ligierten Fragmenten an Kügelchen, wobei jedes Kügelchen an ein einzelnes Fragment angelagert wird;
(f3) Emulgieren der Kügelchen in Wasser-in-01-Mikroreaktoren, wobei jeder Wasser-in-01-Mikroreaktor ein einzelnes Kügelchen umfasst;
(f4) Durchführen einer Emulsions-PCR, um Adapter-ligierte Fragmente an der Oberfläche der Kügelchen zu amplifizieren;
(f5) Auswählen/Anreichern von Kügelchen, die amplifizierte Adapter-ligierte Fragmente enthalten;
(f6) Laden der Kügelchen in Vertiefungen, wobei jede Vertiefung ein einzelnes Kügelchen umfasst, und
(f7) Erzeugen eines Pyrophosphat-Signals.

15. Verfahren gemäß einem der vorstehenden Ansprüche, wobei das Aufbauen von Contigs ferner durch die Verwendung von Nucleotidsequenzen unterstützt wird, die aus anderen Quellen abgeleitet sind, einschließlich, aber nicht beschränkt auf BAC-Endsequenzen, BAC-Shotgunsequenzen, EST-Sequenzen oder ShotgunSequenzen eines ganzen Genoms.

16. Verfahren gemäß einem der vorherigen Ansprüche, wobei das Verfahren zum Reduzieren der Komplexität des Gemisches auf indexierenden Linkern, der Chromatin-Immun-Präzipitation (ChIP) oder gegen konservierte Motive gerichteten PCR-Primern basiert.

## Revendications

1. Procédé de détermination d'une séquence de génome, comprenant les stades dans lesquels :
(a) on se procure un premier sous-jeu du génome en faisant digérer le génome avec au moins une première endonucléase de restriction pour obtenir des fragments de restriction ;
(b) on ligature au moins un adaptateur aux fragments de restriction du premier sous-jeu pour obtenir un premier jeu de fragments de restriction ligaturés à un adaptateur ;
(c) on amplifie, sélectivement ou non sélectivement, le premier jeu de fragments de restriction ligaturés à un adaptateur, en utilisant une première combinaison d'amorces, dans laquelle au moins une première amorce contient une section qui est complémentaire de l'adaptateur et à une partie de la séquence de reconnaissance de l'endonucléase de restriction et qui contient en outre une première séquence sélectionnée à l'extrémité 3' de la séquence d'amorce, la première séquence sélectionnée comprenant de 0 à 10 nucléotides sélectifs pour obtenir un premier sous-jeu de fragments de restriction amplifiés et ligaturés à un adaptateur ;
(d) on répète le stade (c) avec au moins une seconde amorce et/ou d'autres combinaisons d'amorces, dans lequel l'amorce contient une deuxième et/ou une autre séquence sélectionnée différente à son extrémité 3', qui contient le même nombre de nucléotides sélectifs pour obtenir un second sous-jeu et/ou d'autres sous-jeux de fragments de restriction amplifiés et ligaturés à un adaptateur ;
(e) on fragmente chacun des premier, deuxième et/ou autres sous-jeux de fragments de restriction amplifiés et ligaturés à un adaptateur pour produire des première, deuxième et/ou autres banques de séquençage ;
(f) on détermine au moins une partie de la séquence de nucléotides d'au moins une partie des fragments contenus dans chacune des première, deuxième et/ou autres banques ;
(g) on aligne la séquence des fragments dans chacune des première, deuxième et/ou autres banques pour produire des contigus des fragments de restriction amplifiés et ligaturés à un adaptateur dérivés représentant des fractions dispersées du génome ;
(h) on répète les stades (a) à (g) pour au moins une deuxième endonucléase et/ou autres endonucléases de restriction ;
(i) On aligne les contigus obtenus au stade (g) et (h) pour chacune de la deuxième endonucléase et/ou des autres endonucléases de restriction pour obtenir une séquence du génome.

2. Procédé suivant la revendication 1, dans lequel au moins l'une de la première, deuxième et/ou autres endonucléases de restriction est un coupeur rare.

3. Procédé suivant la revendication 1 ou 2, dans lequel au moins l'un de la première, deuxième et/ou autres endonucléases de restriction est un coupeur fréquent.

4. Procédé suivant la revendication 1, 2 ou 3, dans lequel on utilise au moins deux coupeurs rares.

5. Procédé suivant les revendications 1 à 4, dans lequel on utilise un coupeur rare et un coupeur fréquent.

6. Procédé suivant les revendications 1 à 5, dans lequel le procédé d'amplification est la PCR.

7. Procédé suivant les revendications 1 à 6, dans lequel la séquence sélectionnée à l'extrémité 3' de l'amorce contient de 1 à 8 nucléotides sélectionnés.

8. Procédé suivant les revendications 1 à 7, dans lequel la première, deuxième et autre séquence sélectionnée ont le même nombre de nucléotides mais diffère dans la séquence de nucléotides l'une de l'autre dans la séquence sélective placée à l'extrémité 3' de l'amorce.

9. Procédé suivant les revendications 1 à 8, dans lequel on effectue le séquençage par un séquençage Sanger dideoxy.

10. Procédé suivant les revendications 1 à 8, dans lequel on effectue le séquençage sur un support solide, tel qu'une perle.

11. Procédé suivant les revendications 1 à 8, dans lequel le séquençage repose sur le High Throughput Sequencing.

12. Procédé suivant les revendications 1 à 8, dans lequel on effectue le séquençage repose sur le Sequencing-by-Synthesis.

13. Procédé suivant les revendications 1 à 8, dans lequel le séquençage repose sur le Pyrosequencing.

14. Procédé suivant les revendications 1 à 8, dans lequel le séquençage comprend les stades dans lesquels :
(f1) on ligature des adaptateurs de séquençage aux fragments ;
(f2) on séquence avec circularisation des fragments ligaturés à un adaptateur à des perles, chaque perle se circularisant avec un fragment unique ;
(f3) on émulsionne les perles dans des microréacteurs eau dans huile, chaque microréacteur eau dans huile comprenant une perle unique ;
(f4) on effectue une PCR en émulsion pour amplifier des fragments ligaturés à un adaptateur sur la surface des perles ;
(f5) on sélectionne/enrichit des perles contenant des fragments amplifiés et ligaturés à un adaptateur ;
(f6) on charge les perles dans des puits, chaque puits comprenant une perle unique ; et
(f7) on produit un signal pyrophosphate.

15. Procédé suivant l'une quelconques des revendications précédentes, dans lequel on facilite en outre la formation de contigus par l'utilisation de séquences de nucléotides dérivées d'autres sources, y compris, mais sans limitation, des séquences d'extrémité BAC, des séquences au hasard BAC, des séquences EST ou des séquences au hasard de tout le génome.

16. Procédé suivant l'une quelconques des revendications précédentes, dans lequel le procédé de réduction de la complexité du mélange repose sur des segments de liaison d'indexation, une immuno-précipitation à la chromatine (CHIP) ou des amorces de PCR dirigées contre des motifs conservés.
